# EUROPEAN PATENT APPLICATION

(11) **EP 4 714 461 A1**
(43) Date of publication of application: **25.03.2026**
(21) Application number: 24733254.7
(22) Date of filing: 14.05.2024
(51) Int. Cl.: A61K 36/074, A61K 8/00, A61K 9/00, A61K 31/00, A61K 36/07, A61P 17/02, A61P 17/16, A61P 39/00

(54) **USE OF A GANODERMA LUCIDUM EXTRACT AND A HERICIUM ERINACEUS EXTRACT FOR THE TREATMENT OF THE SIDE EFFECTS OF RADIOTHERAPY AND/OR CHEMOTHERAPY ON THE SKIN**

(30) Priority: 19.05.2023 ES 202330388
(71) Applicant: Hifas da Terra, S.L., 36154 Pontevedra (ES)
(72) Inventor: FERNÁNDEZ DE ANA PORTELA, Catalina, 36154 Pontevedra (ES); SINDE STOMPEL, Esteban, 36154 Pontevedra (ES); MAYAN SANTOS, María Dolores, 36154 Pontevedra (ES); FONTELO RODRÍGUEZ, Raúl, 36154 Pontevedra (ES); CARPINTERO FERNÁNDEZ, Paula, 36154 Pontevedra (ES)
(74) Representative: Pons IP
(86) International application number: PCT/ES2024/070290
(87) International publication number: WO 2024/240975

(57) **Abstract**

The present invention relates to a topical composition comprising an extract obtained from *Ganoderma lucidum* and an extract obtained from *Hericium erinaceus.* Furthermore, the present invention relates to a combination of an extract obtained *Ganoderma lucidum* and an extract obtained from *Hericium erinaceus* for use in the treatment and/or prevention of the side effects of radiotherapy and/or chemotherapy on the skin of a subject.

## Description

The present invention is in the field of biomedicine, referring to a topical composition comprising an extract obtained from *Ganoderma lucidum* and an extract obtained from *Hericium erinaceus,* furthermore the present invention relates to a combination of a *Ganoderma lucidum* extract and an extract obtained from *Hericium erinaceus* for use in the treatment and/or prevention of the side effects of radiotherapy and/or chemotherapy on the skin of a subject.

### BACKGROUND OF THE INVENTION

Cancer is one of the leading causes of morbidity and mortality in developed countries. New therapies and combinations have led to increased survival. However, antineoplastic therapies involve many adverse events.

The most common methods for treating cancer include radiotherapy and chemotherapy. Radiotherapy and chemotherapy can damage the skin causing, for example, fibrosis, inflammation, dermatitis, skin fragility, erosions in skin folds, loss of nails or dry skin.

Therefore, there is a need to provide compositions capable of preventing, curing, and/or alleviating the side effects of radiotherapy and/or chemotherapy on the skin. Exposure of the skin to ionising radiation results in a skin reaction to radiation. Immediately after irradiation, cytokine production by skin cells begins and it continues as a cascade throughout all stages of the cutaneous radiation syndrome. Cytokines are important signalling molecules that mediate communicative interactions both locally between different cell types within dermal tissues and at a distance between organs. Scientific studies indicate that the main cytokines in skin cell response to ionising radiation include IL (interleukin)-1, IL-6 and the chemokines IL-8.

The use of extracts originating from fungi for skin care and treatment is described in the state of the art.

Document CN114344360A describes a preparation for skin light injury repair which contains *Ganoderma lucidum* extract.

Mingyi, Y., et al., 2019, Trends in Food Science & Technology, 92, 94-110 describes the use of fungal polysaccharides as nutraceuticals, pharmaceutical products, and cosmetics, highlighting their activity as immunomodulators, anti-tumour agents, and anti-inflammatory agents.

Taofiq, O., et al, 2016, Industrial Crops and Products, 90, 38-48, refers to the use of fungal ingredients to combat skin ageing, hyperpigmentation, and inflammation, said ingredients being used as cosmeceuticals or nutricosmetics as a result of their anti-tyrosinase, anti-hyaluronidase, anti-elastase, and anti-collagenase activities.

Xu, H., International Journal of Biological Macromolecules, 47(1), 33-36 discloses the effects of an *H. erinaceus* polysaccharide extract in improving skin antioxidant enzymes, MMP-1 and TIMP-1 activities, and collagen concentrations.

Document CN105816394 A describes a cream containing *Hericium erinaceus* extract. The cream can repair skin injuries and prevent radiation in order to protect the skin from injuries. The anti-radiation effect is effective against electromagnetic radiation, ultraviolet rays, or microwave radiation.

Document KR20190027178A describes a *Centipeda minima* extract-based cosmetic composition which is fermented with strains including *Hericium erinaceum, Pleurotus ferulae,* Sparassis crispa, *Ganoderma lucidum, Flammulina velutipes, Phellinus linteus,* and/or *Grifola frondosa.*

Document CN108042588A discloses a nutraceutical composition comprising American ginseng root, *Ganoderma lucidum,* and *Hericium erinaceum,* which improves immunity and can be applied after chemotherapy and radiotherapy.

Radiotherapy and chemotherapy can damage the skin. Therefore, there is a need to provide new compositions capable of preventing, treating, curing, and/or alleviating the side effects of radiotherapy and/or chemotherapy in an effective and improved manner, particularly those side effects caused on the skin.

### DESCRIPTION OF THE INVENTION

The authors of the present invention have developed a combination of fungal extracts for use in the treatment and/or prevention of the side effects of radiotherapy and/or chemotherapy on the skin of a subject.

The present inventors have obtained a higher anti-inflammatory activity in vitro in fibroblast cell lines by combining *Hericium erinaceus* carpophore extract and *Ganoderma lucidum* carpophore extract, in comparison to these extracts individually (see Table 1 of the examples). Thus, based on the results obtained, the inventors have therefore proven an improved anti-inflammatory effect of the combination of *Hericium erinaceus* and *Ganoderma lucidum* extracts, with the use of said combination being of interest for the treatment and/or prevention of inflammatory conditions such as the side effects of radiotherapy and/or chemotherapy on the skin of a subject.

Preparations containing an extract obtained from the carpophore of *Ganoderma lucidum* or an extract obtained from the carpophore of *Hericium erinaceus* are known in the art. However, an extract obtained from *Ganoderma lucidum* in combination with an extract obtained from *Hericium erinaceus* has not been used previously for the prevention and/or treatment of the side effects of radiotherapy and/or chemotherapy on the skin. Furthermore, the present strategy to combat the side effects of radiotherapy and/or chemotherapy on the skin provides several advantages. By means of administering an extract obtained from *Ganoderma lucidum* and an extract obtained from *Hericium erinaceus* directly at the site of action, improved local immunity is achieved. Side effects can be minimised because by combining the extracts and administering them topically, the therapeutically effective dose can be adjusted more easily to achieve the desired effect, while an optimal dose-response can be achieved at the same time.

Therefore, in a first aspect, the present invention relates to a topical composition, hereinafter "the composition of the invention", which comprises an extract obtained from *Ganoderma lucidum* and an extract obtained from *Hericium erinaceus.*

In the present invention, the term "topical composition" refers to a composition suitable for application on the external tissues of a subject such as the skin, scalp, eyelashes, eyebrows, nails, mucous membranes, and hair, preferably the skin. Therefore, as understood by the person skilled in the art, the composition of the invention is formulated for topical administration.

*Ganoderma lucidum* is a basidiomycete of the *Ganodermataceae* family and contains a large number of bioactive compounds, especially β-glucan polysaccharides, which are responsible for the anti-tumour, immunomodulatory, and antioxidant activities of this species.

The term "extract obtained from *Ganoderma lucidum"* refers to one or more compounds, substance or substances, sample, concentrate, or isolated product, which has been obtained, derived, or extracted from the fungus *Ganoderma lucidum.* This includes complete *Ganoderma lucidum* extracts, but also pure or semi-pure preparations of the biologically active compounds obtained from *Ganoderma lucidum,* preferably obtained from the carpophore of *Ganoderma lucidum,* as well as from the dust of the carpophore. *Ganoderma lucidum* extract can be obtained through physical and/or chemical extraction methods. *Ganoderma lucidum* extract also preferably includes combinations of other known components and/or active compounds which are combined to substantially imitate the composition and/or the activity of a natural *Ganoderma lucidum* extract. In the present invention, the terms "extract obtained from *Ganoderma lucidum" and "Ganoderma lucidum* extract" can be used interchangeably.

**In** a preferred embodiment, the *Ganoderma lucidum* extract comprises β-glucans, ergosterol, and ganoderic acids.

**In** a more preferred embodiment, the *G. lucidum* extract comprises:
- between 524 mg/g and 641 mg/g of β-glucans;
- between 376 µg/g and 460 µg/g of ergosterol; and
- between 4.30 µg/g and 5.30 µg/g of ganoderic acids.

**In** a more preferred embodiment, the *G. lucidum* extract comprises:
- 583 mg/g of β-glucans;
- 418 µg/g of ergosterol; and
- 4.81 µg/g of ganoderic acid.

The quantification of β-glucans, ergosterol, and ganoderic acid can be performed following standardised methodologies. β-glucans were quantified using a β-glucan enzyme kit (Megazyme, Neogen). Ergosterol is quantified by high-performance liquid chromatography coupled with mass spectrometry (HPLC-MS) following the methodology described by Montgomery et al. Soil Biology and Biochemistry. 2020; 32: 1207-1217, and ganoderic acid is quantified by HPLC-MS following the methodology described by Qui et al. Front Farmacol. 2012; 3: 85.

*Hericium erinaceus* is a basidiomycete of the *Hericiaceae* family, is considered a natural prebiotic which helps in microbiota recovery, and contains a large number of bioactive compounds, particularly the β-glucan polysaccharides, which are responsible for the anti-cancer, immunomodulatory, antioxidant, neurostimulatory, and neuroprotective activities of this species.

The term "extract obtained from *Hericium erinaceus"* refers to one or more compounds, substance or substances, sample, concentrate, or isolated product, which is obtained, derived, or extracted from the fungus *Hericium erinaceus.* This includes complete *H*. *erinaceus* extracts, but also pure or semi-pure preparations of the biologically active compounds obtained from *H. erinaceus,* preferably obtained from the carpophore of *H*. *erinaceus,* as well as from the dust of the carpophore. *Hericium erinaceus* extract can be obtained through physical and/or chemical extraction methods. *Hericium erinaceus* extract also preferably includes combinations of other known components and/or active compounds which are combined to substantially imitate the composition and/or the activity of a natural *H. erinaceus* extract. In the present invention, the terms "extract obtained from *Hericium erinaceus"* and *"Hericium erinaceus* extract" can be used interchangeably.

In a preferred embodiment, the *H. erinaceus* extract comprises β-glucans, γ-aminobutyric acid, ergosterol, and hericenone c.

In a more preferred embodiment, the *H. erinaceus* extract comprises:
- between 220 mg/g and 268 mg/g of β-glucans;
- between 46 µg/g and 56.32 µg/g of γ-aminobutyric acid;
- between 313.39 µg/g and 382.80 µg/g of ergosterol; and
- between 0.18 µg/g and 0.22 µg/g of hericenone c.

In a more preferred embodiment, the *H. erinaceus* extract comprises:
- 244 mg/g of β-glucans;
- 51.2 µg/g of γ-aminobutyric acid;
- 348.21 µg/g of ergosterol; and
- 0.2 µg/g of hericenone c.

The quantification of β-glucans, γ-aminobutyric acid, ergosterol, and hericenone c can be performed following standardised methodologies. β-glucans are quantified using a β-glucan enzyme kit (Megazyme). γ-aminobutyric acid is quantified by HPLC following the methodology described by Gong et al. J. Inst. Brew.2017; 123: 417-422; ergosterol is quantified by HPLC-MS following the methodology described by Montgomery et al. Soil Biology and Biochemistry. 2020; 32: 1207-1217, and hericenone c is quantified by HPLC following the methodology described by Lee et al. Journal of Ethnopharmacology. 2016; 184: 219-225.

In the present invention, the extract obtained from *Ganoderma lucidum* and the extract obtained from *Hericium erinaceus* are referred to as "the extracts of the invention".

In a preferred embodiment of the composition of the invention, the extracts obtained from *Ganoderma lucidum* and *Hericium erinaceus* are obtained from the carpophore or mycelium of said fungi, preferably from the carpophore. Therefore, in a preferred embodiment, the composition of the invention comprises an extract obtained from the carpophore of *Ganoderma lucidum* and an extract obtained from the carpophore of *Hericium erinaceus.*

Therefore, in preferred embodiments, the concentration of β-glucans, ergosterol, and ganoderic acids in the *Ganoderma lucidum* extract; and the concentration of β-glucans, γ-aminobutyric acid, ergosterol, and hericenone c in the *Hericium erinaceus* extract, described in the preceding embodiments, correspond to the concentrations of said compounds in extracts obtained from the carpophore of *Ganoderma lucidum* and from the carpophore of *Hericium erinaceus,* respectively.

The term "carpophore" refers to the reproductive part of the fungus, also referred to as fruiting body, sporocarp, or mushroom.

The term "mycelium" refers to the mass of hyphae which form the vegetative part of a fungus.

In another preferred embodiment, the composition of the invention comprises the *H*. *erinaceus* extract in a concentration comprised between 0.005% and 25%, in % m/m, and the *G. lucidum* extract in a concentration comprised between 0.005% and 25%, in % m/m, depending on the form in which said composition is presented, for example, depending on whether the composition is in the form of a gel, cream, or ointment. The % refer to the % m/m of each of the extracts with respect to the total composition.

**In** another even more preferred embodiment of the invention, the composition of the invention comprises the *H. erinaceus* extract in a concentration comprised between 0.25% m/m and 3% m/m, preferably in a concentration of 0.5% m/m, 1% m/m, 1.5% m/m, 2% m/m, or 2.5% m/m, more preferably 0.5% m/m, and the G. *lucidum* extract in a concentration comprised between 0.25% m/m and 3% m/m, preferably in a concentration of 0.5% m/m, wherein the % refers to the % m/m of each of the extracts with respect to the total composition in which they are found.

The term "% m/m" refers to the percentage by mass of the extract, i.e., a measurement of concentration indicating the mass of the solute (e.g., *H. erinaceus* extract/G. *lucidum* extract) per 100 mass units of the composition.

Examples of methods for obtaining the extracts of the invention include, but are not limited to, pressing, soaking, soaking under stirring, ultrasound-assisted extraction, percolation or re-percolation, vapour-entrainment distillation, hydrodistillation, decantation, filtration, evaporation, and/or chromatography. Preferably, the extraction method of the invention is percolation.

**In** a particular embodiment, the *Ganoderma lucidum* extract, particularly *Ganoderma lucidum* carpophore extract, or the *Hericium erinaceus* extract, particularly *Hericium erinaceus* carpophore extract, is obtained by a method, hereinafter "obtention method of the invention", which comprises the following steps:
(i) mixing the entire carpophore or portions of carpophore, preferably having a size smaller than 5 cm, with an aqueous solution and/or alcohol, preferably in a solid:liquid proportion of 1:5 to 1:40;
(ii) subjecting the heterogeneous mixture obtained in (i) to a temperature of between 20°C and 70°C for between 30 and 60 minutes, obtaining an infusion of the mixture obtained in (i); and
(iii) percolating or filtering the infusion obtained in (ii), obtaining carpophore extract in the liquid fraction or solution.

The extracts of the invention can be obtained from the entire carpophore or carpophore cut into small pieces or portions having a size smaller than 5 centimetres (cm), preferably smaller than 4 cm, more preferably smaller than 3 cm, or smaller than 2 cm. **In** another particular embodiment of the obtention method of the invention, the carpophore extract is obtained from portions of carpophore having a size smaller than 1 cm.

**In** another more particular embodiment of the obtention method of the invention, the carpophore extract is obtained by mixing the entire carpophore or portions of carpophore with a solvent, preferably wherein the solvent is selected from the list consisting of: an aqueous solution (more preferably water), an alcohol (more preferably ethanol, methanol, butanol, propanol-2, or pentanol), propane, butane, ethyl acetate, carbon dioxide, acetone, nitrous oxide, or a mixture thereof.

**In** another more particular embodiment of the obtention method of the invention, the carpophore extract is obtained by mixing the entire carpophore or portions of carpophore with an aqueous solution and alcohol, preferably water and/or alcohol.

In another still more particular embodiment of the obtention method of the invention, the carpophore extract is obtained by mixing the entire carpophore or portions of carpophore with water and/or ethanol, more preferably with a mixture of water and ethanol.

When a mixture of ethanol and water is used, the ethanol is preferably in a proportion of between 10% by volume and 60% by volume (wherein water would be in a proportion of between 90% and 40% by volume, respectively), more preferably between 20% and 60% by volume (wherein water would be in a proportion of between 80% and 40% by volume, respectively), even more preferably between 40% and 60% by volume (wherein water would be in a proportion of between 60% and 40% by volume, respectively).

In another more particular embodiment of the obtention method of the invention, the carpophore extract is obtained by mixing the entire carpophore or portions of carpophore with water and ethanol in a water:ethanol proportion of 50% and 50% by volume, i.e., in a water:ethanol proportion of 1:1.

Particularly, to obtain the carpophore extract, the proportion of the entire carpophore or of portions of carpophore (solid) in the solvent (liquid), i.e., the solid:liquid proportion, is from 1:5 to 1:40. In another particular embodiment, the proportion of the entire carpophore or portions of carpophore (solid) in the solvent (liquid) is from 1:10 to 1:30, preferably 1:20.

The time needed for extraction is usually adapted to the starting fungal material, the extraction method, the extraction temperature, the ratio between the solvent and the fungal material, and the like.

Once the mixture of the entire carpophore or portions of carpophore and the solvent is obtained, said mixture is subjected to a temperature of between 20 and 70°C for between 30 and 60 minutes (step (ii)), obtaining an infusion of the obtained mixture.

In another more particular embodiment of the obtention method of the invention, the mixture of the entire carpophore or portions of carpophore and the solvent is subjected to a temperature of between 30 and 60°C, preferably between 35 and 55°C, for between 35 and 55 minutes, preferably between 40 and 50 minutes.

In another more particular embodiment of the obtention method of the invention, the mixture of the entire carpophore or portions of carpophore and the solvent is subjected to a temperature of 36, 37, 38, 39, 40, 41, 42, 43, 44, 45, 46, 47, 48, 49, 50, 51, 52, 53, or 54°C for 41, 42, 43, 44, 45, 46, 47, 48, or 49 minutes.

In another even more preferred embodiment of the obtention method of the invention, the mixture of the entire carpophore or portions of carpophore and the solvent is subjected to a temperature of 50°C for 45 minutes.

Optionally, the extraction step can be repeated on the same solid under the same conditions described above, i.e., optionally step (ii) can be repeated at least one time.

After the extraction (step ii), the mixture of solvent and extracted compounds that forms after the solvent comes into contact with the fungal material is separated by means of solid-liquid filtration of the fungal material using methods such as percolation, obtaining the carpophore extract of the invention in the liquid fraction.

The term "percolation" refers to the action of a liquid penetrating, passing, or moving through the pores of a body or environment, i.e., the slow passage of fluids through porous materials. Synonyms of this term in the state of the art are filtration and leaching processes.

The bioactive compounds present in the extract, mainly phenolic compounds, usually have a limited shelf life against oxidation. Therefore, additional drying of the extract (in the liquid fraction) can optionally be applied to extend its shelf life. One of the methods that can be applied for drying bioactive extracts is spray drying.

Therefore, in another more preferred embodiment of the obtention method of the invention, the method for obtaining the carpophore extract further comprises a step (iv) of spray drying the solution obtained in (iii) at a temperature between 135°C and 195°C. In another still more particular embodiment, the temperature of step (iv) is between 145°C and 185°C, preferably between 165°C and 175°C.

An encapsulating agent, for example, maltodextrin, can be added during spray drying.

Therefore, in another more preferred embodiment, the obtention method of the invention further comprises a step (v) of adding an encapsulating agent, preferably the encapsulating agent is in a proportion of between 1% m/m and 50% m/m (wherein the extracts would be in a proportion of between 99% m/m and 50% m/m, respectively), more preferably between 20% m/m and 40% m/m (wherein the extracts would be in a proportion of between 80% and 60% m/m, respectively), even more preferably between 25% m/m and 35% m/m (wherein the extracts would be in a proportion of between 75% m/m and 65% m/m, respectively).

In another more particular embodiment of the method of the invention, the proportion of the combination of extracts:encapsulating agent is 71.4% m/m and 28.57% m/m, i.e., a proportion of extracts:encapsulating agent of 4:14.

The term "encapsulating agent" refers to a substance and/or substances which prevent the ingredient that is subjected to spray drying from agglomerating, caking, and sticking, increasing its stability and improving its bioavailability. Examples of encapsulating agents include, but are not limited to, maltodextrin, starch and derivatives, corn syrups, sucrose, cyclodextrins, carboxymethylcellulose, methylcellulose, ethylcellulose, nitrocellulose, acetylcellulose, gum arabic, sodium alginate, carrageenan, mono- and diglycerides, whey, zein, gluten, and/or casein.

In another more preferred embodiment, the method for obtaining the extract of the invention further comprises a step (v) of adding an encapsulating agent, wherein the encapsulating agent is maltodextrin.

In a preferred embodiment, the composition of the invention is in liquid or semisolid form, preferably in the form of a gel, cream, or ointment.

The composition of the invention can be formulated (i) for pharmaceutical administration, i.e., alone or as part of pharmaceutical products that are administered to a subject, or (ii) for administration as cosmetics to a subject, i.e., alone or as part of cosmetic products. Therefore, in a particular embodiment, the composition of the invention is a pharmaceutical composition or a cosmetic composition, hereinafter "pharmaceutical composition of the invention" and "cosmetic composition of the invention", respectively.

The term "pharmaceutical composition" refers to a set, mixture, combination of components or substances comprising the extracts of the invention in any concentration, and which improves the subject's state of health or reduces the risk of disease. Said pharmaceutical composition can be a medicinal product. However, the term "pharmaceutical composition" also encompasses health products, provided that the "health product" is intended for use on subjects, preferably humans, separately or in combination with other products, for one of the following specific medical purposes: prevention, treatment, or alleviation of a disease, or treatment, alleviation, or compensation of an injury or disability and its primary mechanism of action is not pharmacological, metabolic, or immunological.

In the present invention, the term "cosmetic composition" refers to a set, mixture, combination of components or substances comprising the extracts of the invention in any concentration intended to be brought into contact with the different external parts of a subject's body (epidermis, hair system, nails, lips, and external genital organs) for the sole or main purpose of cleaning them, perfuming them, modifying their appearance, and/or correcting body odour, and/or protecting or maintaining them in good condition.

In another preferred embodiment, the composition of the invention further comprises pharmaceutically and cosmetically acceptable vehicle and/or excipients.

Therefore, in another preferred embodiment, the pharmaceutically or cosmetically acceptable vehicle and/or excipient of the composition of the invention is water, chitosan, hydroxypropyl methylcellulose, lactic acid, sodium hyaluronate, cranberry flavouring, citric acid, sorbic acid, sodium benzoate, organic rice extract, rice fibre, rice protein, silicon dioxide, calcium carbonate, dicalcium phosphate, sorbitol, sucralose, xylitol, and/or combinations thereof.

In another more preferred embodiment, the pharmaceutically or cosmetically acceptable vehicle and/or excipient of the composition of the invention is lactic acid, sodium hyaluronate, cranberry flavouring, citric acid, sorbic acid, sodium benzoate, chitosan, water and/or combinations thereof.

As understood by the person skilled in the art, the composition of the invention may comprise at least one bioactive component (active substance, active ingredient, or therapeutic agent) such as, for example, prebiotics, probiotics, vitamins, minerals, plant or fungal products, and/or drugs.

As have been proven by the researchers, the combination comprising a *Hericium erinaceus* extract, preferably an extract obtained from the carpophore of *Hericium erinaceus,* and a *Ganoderma lucidum* extract, preferably an extract obtained from the carpophore of *Ganoderma lucidum,* hereinafter "combination of the invention", has therapeutic properties, such that in another aspect, the present invention relates to the combination of the invention for use as a medicinal product, wherein said medicinal product is administered topically, the "first medical use" of the invention.

The term "medicinal product" refers to any substance or combination of substances which is presented as having properties for the treatment or prevention of diseases in a subject, or may be used in or administered to a subject for the purpose of restoring, correcting, or modifying physiological functions by exerting a pharmacological, immunological, or metabolic action, or for the purpose of making a medical diagnosis.

In a preferred embodiment of the first medical use of the invention, the *Hericium erinaceus* and *Ganoderma lucidum* extracts are part of the topical composition of the invention.

In the present invention, the term "subject" refers to a human or any eukaryotic, heterotrophic, and multicellular non-human organism, such as a non-human animal. Examples of non-human animals include, but are not limited to, non-human mammals such as non-human primates, horses, sheep, goats, pigs, dogs, cows, or cats.

The present inventors have surprisingly found that the combination comprising an extract obtained from *Hericium erinaceus,* and an extract obtained from *Ganoderma lucidum* has anti-inflammatory effects which are beneficial for the treatment and/or prevention of side effects associated with radiotherapy and/or chemotherapy.

Specifically, the inventors found that the administration of the *Ganoderma lucidum* carpophore extract in combination with the *Hericium erinaceus* carpophore extract significantly reduced the mRNA expression of proinflammatory cytokines IL-1 β, IL-6, and IL-8, particularly IL-8, in comparison to the administration of the fungal extracts individually. Therefore, the inventors have proven an improved anti-inflammatory effect of the combination of *Ganoderma lucidum* and *Hericium erinaceus* extracts with respect to said extracts individually.

Therefore, in another aspect, the present invention relates to the use of an extract obtained from *Ganoderma lucidum,* preferably an extract obtained from the carpophore of *Ganoderma lucidum,* and an extract obtained from *Hericium erinaceus,* preferably an extract obtained from the carpophore of *Hericium erinaceus,* for the elaboration of a medicinal product for the treatment and/or prevention of side effects associated with radiotherapy and/or chemotherapy in a subject, preferably the side effects associated with radiotherapy and/or chemotherapy show up on the skin of the subject.

**In** another aspect, the present invention relates to the combination of the invention, i.e., a combination comprising an extract obtained from *Ganoderma lucidum* and an extract obtained *Hericium erinaceus,* for use in the treatment and/or prevention of the side effects of radiotherapy and/or chemotherapy on the skin of a subject, hereinafter "the second medical use of the invention".

The terms "extract obtained from *Ganoderma lucidum",* "extract obtained from *Hericium erinaceus",* and "subject" have been explained above, and both said terms and their preferred embodiments are applicable to the present aspect of the invention.

**In** the present invention, the term "treatment" refers to combating the effects caused by a disease or pathological condition of interest in a subject (preferably a mammal, and more preferably a human) which includes:
(i) inhibiting the disease or pathological condition, i.e., stopping its development;
(ii) alleviating the disease or pathological condition, i.e., causing the regression of the disease or pathological condition or its symptomatology;
(iii) stabilising the disease or pathological condition.

**In** the present invention, the term "prevention" refers to the avoidance of the onset of the disease, i.e., avoiding the occurrence of the disease or pathological condition in a subject (preferably mammal, and more preferably a human), particularly when said subject is predisposed to the pathological condition.

The terms "side effect" or "adverse reaction to medicinal products", as used interchangeably herein, refer to any harmless and unintended response to a medicinal product that occurs at doses normally applied to a subject (preferably mammal, and more preferably a human) for the prophylaxis, diagnosis, or treatment of diseases and/or symptoms. Specifically, the side effects of radiotherapy and/or chemotherapy on the skin include, but are not limited to, fibrosis, inflammation, dermatitis, skin fragility, erosions in the skin folds, loss of nails, or dry skin.

The term "radiotherapy" refers to a form of treatment based on the use of ionising radiations. Said ionising radiations comprise X-rays or radioactivity which includes gamma rays and alpha particles.

The term "chemotherapy" refers to a form of treatment based on the administration of chemical substances for the treatment of a disease, particularly cancer. Examples of chemotherapeutic agents which may cause side effects on the skin of subjects to whom they are administered include, but are not limited to, doxorubicin, cyclophosphamide, thiotepa, daunorubicin, doxorubicin, epirubicin, 5-fluorouracil, methotrexate, 6-mercaptopurine, docetaxel, paclitaxel, vinblastine, vincrastine, vincristine, actinomycin, bleomycin, mitomycin, etoposide, or teniposide.

**In** a preferred embodiment of the second medical use of the invention, the side effect of radiotherapy and/or chemotherapy is selected from the list consisting of: fibrosis, inflammation, dermatitis, skin fragility, erosions in skin folds, loss of nails, and/or dry skin. Preferably, the side effect of radiotherapy and/or chemotherapy is selected from the list consisting of skin fragility, dermatitis, and/or erosions in skin folds.

In a preferred embodiment of the second medical use of the invention, the combination of extracts comprises an extract obtained from the carpophore of *Ganoderma lucidum* and an extract obtained from the carpophore of *Hericium erinaceus.*

In another preferred embodiment of the second medical use of the invention, the extracts of the combination of the invention are administered to the subject simultaneously, separately, or sequentially.

In a preferred embodiment of the second medical use of the invention, the route of administration is topical.

In another preferred embodiment of the second medical use of the invention, both the extract obtained from *Ganoderma lucidum* and the extract obtained from *Hericium erinaceus* are administered to the subject in a therapeutically effective dose, which may vary over a wide range. In the present invention, the expressions "therapeutically effective dose" or "therapeutically effective amount", used interchangeably herein, refer to that amount or dose of the extracts obtained from *Ganoderma lucidum* and *Hericium erinaceus* which, when administered to a subject, preferably to a mammal, and more preferably to a human, is sufficient to cause the prevention and/or treatment of the side effects associated with radiotherapy and/or chemotherapy on the skin. The therapeutically effective amount will vary, for example, according to the metabolic stability and duration of action of the extracts; the age, body weight, general health condition, sex, and diet of the subject; the mode and time of administration; the excretion rate, the combination with other drugs; the severity of the particular disease or pathological condition of said subject. It is routine practice for a person skilled in the art, for example, a medical specialist, to determine the therapeutically effective dose for each subject, preferably a human, according to his or her own knowledge and the physiological variables or conditions of the subject.

Given that the extracts of the combination of the invention can be administered simultaneously, separately, or sequentially, in a preferred embodiment, the extracts of the combination of the invention are comprised in one and the same composition or in different compositions (i.e., each of the extracts is in a different composition). Both in different compositions and in one and the same composition, the *Ganoderma lucidum* extract is in an amount of between 0.005% m/m and 25% m/m, and wherein the *Hericium erinaceus* extract is in an amount of between 0.005% m/m and 25% m/m, either in different compositions or in the same composition, wherein the % refers to the % m/m of each of the extracts with respect to the total composition in which they are found.

In another even more preferred embodiment of the second medical use of the invention, both in different compositions and in one and the same composition, the *Ganoderma lucidum* extract is in an amount of between 0.25% m/m and 3% m/m, preferably in a concentration of 0.5% m/m, 1% m/m, 1.5% m/m, 2% m/m, or 2.5% m/m, more preferably 0.5% m/m, and wherein the *Hericium erinaceus* extract is in an amount of between 0.25% m/m and 3% m/m, preferably in a concentration of 0.5% m/m, 1% m/m, 1.5% m/m, 2% m/m, or 2.5% m/m, more preferably 0.5% m/m, wherein the % refers to the % m/m of each of the extracts with respect to the total composition in which they are found.

In a preferred embodiment of the second medical use of the invention, the composition or compositions comprising the extracts obtained from *Ganoderma lucidum* and *Hericium erinaceus* are pharmaceutical composition or compositions. In another preferred embodiment of the second medical use of the invention, said pharmaceutical composition or compositions further comprise a pharmaceutically acceptable vehicle and/or excipient.

Therefore, in another preferred embodiment of the second medical use of the invention the pharmaceutically acceptable vehicle and/or excipient is lactic acid, sodium hyaluronate, citric acid, sorbic acid, sodium benzoate, chitosan, water, and/or combinations thereof.

The composition or compositions of the second medical use of the invention can be formulated for pharmaceutical administration. Therefore, in a preferred embodiment of the second medical use of the invention, the composition or compositions are in liquid or semisolid form, preferably formulated in the form of a gel, cream, or ointment.

The combination of the extracts of the invention can be applied on the area affected by the side effects of radiotherapy and/or chemotherapy, for example, the skin of the subject. Therefore, the combination of the extracts of the invention can be administered in a preventive manner on the area in risk of being affected by the side effects of radiotherapy and/or chemotherapy. The combination, for example, comprised in the composition of the invention, can be applied on said area by gentle rubbing for several minutes so that the skin can absorb the active component. For example, but without limitation, the composition of the invention can be applied on the skin affected by or in risk of being affected by the side effects of radiotherapy and/or chemotherapy, before or after radiotherapy and/or chemotherapy treatment.

Therefore, in a preferred embodiment of the second medical use of the invention, the combination of the invention is administered to the subject before or after radiotherapy and/or chemotherapy treatment.

**In** another preferred embodiment of the second medical use of the invention, the composition comprising the extracts obtained from *Ganoderma lucidum* and *Hericium erinaceus* is the composition of the invention.

Chemotherapy or radiotherapy are two of the main treatment strategies for subjects who have cancer. Therefore, in a preferred embodiment of the second medical use of the invention, the subject has or is at risk of having cancer.

The term "cancer" refers to a pathology or group of pathologies characterised by an uncontrolled growth of cells in the individual. Depending on the tissue where the uncontrolled growth originates, examples of cancer include, but are not limited to, breast cancer, ovarian cancer, endometrial cancer, cervical cancer, lymphoid cancer, or brain cancer.

Therefore, in a preferred embodiment of the second medical use of the invention, the cancer is selected from a group comprising: breast cancer, prostate cancer, ovarian cancer, endometrial cancer, cervical cancer, lymphoid cancer, brain cancer, head and neck cancer, leukaemia, lung cancer, colorectal cancer, thyroid cancer, renal cancer, adrenal cancer, liver cancer, stomach cancer, intestinal cancer, kidney cancer, multiple myeloma, non-small cell lung cancer, pancreatic cancer, glioblastoma, melanoma, colon cancer, lymphomas, and/or neuroendocrine tumours. More preferably, the cancer or tumour is breast cancer.

In a preferred embodiment of the second medical use of the invention, the subject is a human or a non-human mammal.

In another aspect, the present invention relates to the non-therapeutic cosmetic use of a combination comprising an extract obtained from *Ganoderma lucidum* and an extract obtained from *Hericium erinaceus,* wherein said combination is administered topically to a subject, hereinafter "cosmetic use of the invention". Cosmetic use refers to preservation of the integrity and/or improvement of the appearance of the skin, hair, and/or mucous membranes of said subject.

The terms "extract obtained from *Ganoderma lucidum",* "extract obtained from *Hericium erinaceus",* and "subject" have been explained above, and both said terms and their preferred embodiments are applicable to the present aspect of the invention.

In a preferred embodiment of the cosmetic use of the invention, the combination of extracts comprises an extract obtained from the carpophore of *Ganoderma lucidum* and an extract obtained from the carpophore of *Hericium erinaceus.*

In a preferred embodiment of the cosmetic use of the invention, the *Ganoderma lucidum* and *Hericium erinaceus* extracts are administered to the subject simultaneously, separately, or sequentially.

In another preferred embodiment of the cosmetic use of the invention, both the extract obtained from *Ganoderma lucidum* and the extract obtained from *Hericium erinaceus* are administered to the subject in a cosmetically effective dose, which may vary over a wide range. In the present invention, the expressions "cosmetically effective dose" or "cosmetically effective amount", used interchangeably herein, refer to that amount or dose of the extracts obtained from *Ganoderma lucidum* and *Hericium erinaceus* which, when administered to a subject, preferably to a mammal, and more preferably to a human, is sufficient to preserve the integrity and/or improve the appearance of the skin, hair, and/or mucous membranes of said subject.

In a preferred embodiment of the cosmetic use of the invention, the *Ganoderma lucidum* and *Hericium erinaceus* extracts are comprised in one and the same composition or in different compositions (i.e., each of the extracts is in a different composition).

Therefore, in another preferred embodiment of the cosmetic use of the invention, both in different compositions and in one and the same composition, the *Ganoderma lucidum* extract is in an amount of between 0.005% m/m and 25% m/m, and wherein the *Hericium erinaceus* extract is in an amount of between 0.005% m/m and 25% m/m, either in different compositions or in the same composition, wherein the % refers to the % m/m of each of the extracts with respect to the total composition in which they are found.

In another even more preferred embodiment of the cosmetic use of the invention, both in different compositions and in one and the same composition, the *Ganoderma lucidum* extract is in an amount of between 0.25% m/m and 3% m/m, preferably in a concentration of 0.5% m/m, 1% m/m, 1.5% m/m, 2% m/m, or 2.5% m/m, more preferably 0.5% m/m, and wherein the *Hericium erinaceus* extract is in an amount of between 0.25% m/m and 3% m/m, preferably in a concentration of 0.5% m/m, 1% m/m, 1.5% m/m, 2% m/m, or 2.5% m/m, more preferably 0.5% m/m, wherein the % refers to the % m/m of each of the extracts with respect to the total composition in which they are found.

In a preferred embodiment of the cosmetic use of the invention, the composition or compositions comprising the extracts obtained from *Ganoderma lucidum* and *Hericium erinaceus* are cosmetic compositions, wherein said cosmetic composition or compositions further comprise a cosmetically acceptable vehicle and/or excipient.

In another preferred embodiment of the cosmetic use of the invention, the composition or compositions are in liquid or semisolid form.

In another preferred embodiment of the cosmetic use of the invention, the composition or compositions are formulated in the form of a gel, cream, ointment.

In another preferred embodiment of the cosmetic use of the invention, the extracts obtained from *Ganoderma lucidum* and *Hericium erinaceus* are comprised in a cosmetic composition, preferably the composition of the invention.

In another preferred embodiment of the cosmetic use of the invention, the subject is a human or a non-human mammal.

In preferred embodiments of the composition of the invention, the first medical use of the invention, the second medical use of the invention, or the cosmetic use of the invention, the *Ganoderma lucidum* and *Hericium erinaceus* extracts are obtained by means of the obtention method of the invention.

In preferred embodiments of the first medical use of the invention, the second medical use of the invention, or the cosmetic use of the invention, the *Ganoderma lucidum* extract comprises the composition of β-glucans, ergosterol, and ganoderic acids according to any one of the preferred embodiments of the composition of the invention; and the *Hericium erinaceus* extract comprises the composition of β-glucans, γ-aminobutyric, ergosterol, and hericenone c according to any one of the preferred embodiments of the composition of the invention.

### EXAMPLES

The invention is illustrated below by means of assays carried out by the inventors which reveal the effectiveness of the product of the invention.

### Example 1. Obtaining Hericium erinaceus and Ganoderma lucidum extracts.

### Process for obtaining Hericium erinaceus carpophore extract.

The method for preparing a *Hericium erinaceus* fungal extract from carpophore cut to a size smaller than 1 cm comprises:
a) extracting a fungal material by percolation using water as a solvent and 50% ethanol with a solid:liquid proportion of 1:20 for 45 minutes at a temperature of 50°C. After extraction, the mixture of solvent and extracted compounds that forms after the solvent comes into contact with the fungal material is separated by means of solid-liquid filtration of the fungal material.
b) drying the extract by spray drying. During spray-drying, moisture evaporation occurs at a temperature between 135°C and 195°C. During spray-drying, maltodextrin is added as an encapsulating agent at an extract:encapsulating agent ratio of 4:14.

The *Hericium erinaceus* carpophore extract obtained contains 244 mg/g of betaglucans, 51.2 µg/g of γ-aminobutyric acid (GABA), 348.2 µg/g of ergosterol, and 0.2 µg/g of hericenone C.

The quantification of β-glucans, γ-aminobutyric acid, ergosterol, and hericenone c was performed following standardised methodologies. β-glucans were quantified using a β-glucan enzyme kit (Megazyme). γ-aminobutyric acid was quantified by HPLC following the methodology described by Gong et al. J. Inst. Brew.2017; 123: 417-422; ergosterol was quantified by HPLC-MS following the methodology described by Montgomery et al. Soil Biology and Biochemistry. 2020; 32: 1207-1217, and hericenone c was quantified by HPLC following the methodology described by Lee et al. Journal of Ethnopharmacology. 2016; 184: 219-225.

### Process for obtaining Ganoderma lucidum carpophore extract.

The method for preparing a *Ganoderma lucidum* fungal extract from carpophore cut to a size smaller than 1 cm comprises:
a) extracting a fungal material by percolation using water as a solvent and 50% ethanol with a solid:liquid proportion of 1:20 for 45 minutes at a temperature of 50°C. After extraction, the mixture of solvent and extracted compounds that forms after the solvent comes into contact with the fungal material is separated by means of solid-liquid filtration of the fungal material.
b) drying the extract by spray drying. During spray-drying, moisture evaporation occurs at a temperature between 135°C and 195°C. During spray-drying, maltodextrin is added as an encapsulating agent at an extract:encapsulating agent ratio of 4:14.

The *Ganoderma lucidum* carpophore extract obtained contains 582.8 mg/g of betaglucans, 418 µg/g of ergosterol, and 4.81 µg/g of ganoderic acid.

The quantification of β-glucans, ergosterol, and ganoderic acids was performed following standardised methodologies. β-glucans were quantified using a β-glucan enzyme kit (Megazyme). Ergosterol was quantified by high-performance liquid chromatography coupled with mass spectrometry (HPLC-MS) following the methodology described by Montgomery et al. Soil Biology and Biochemistry. 2020; 32: 1207-1217, and ganoderic acid was quantified by HPLC-MS following the methodology described by Qui et al. Front Farmacol. 2012; 3: 85.

### Example 2. Isolation of primary fibroblasts, cell culture, and treatments.

Primary cells isolated from patients were used in this study. Cells were cultured in Petri dishes having a diameter of 10 centimetres (cm) in Dulbecco's Modified Eagle Medium (DMEM, Lonza) complemented with 10% foetal bovine serum (FBS) and 1% penicillin/streptomycin (P/S). Cells were incubated in culture ovens at 37°C and 5% CO2. Dermal fibroblasts were cultured for 24 h. Subsequently, the *Ganoderma lucidum* carpophore extract was added at a concentration of 10 ng/ml, the *Hericium erinaceus* carpophore extract was added at a concentration of 10 ng/ml, and a mixture of *Ganoderma lucidum* carpophore extract and *Hericium erinaceus* carpophore extract was added at a concentration of 10 ng/ml, the mixture of *Ganoderma lucidum* carpophore extract and *Hericium erinaceus* carpophore extract is in a proportion of 1:1. The extracts were diluted in water to the mentioned concentrations. Fibroblasts to which no compound whatsoever was added were used as a control. After 24 hours, samples were collected and the mRNA expression levels of the cytokines IL-1β, IL-6, and IL-8 were analysed following standardised methodologies. RNA extraction was carried out using Tryzol^{®} Reagent (Invitrogen) following the protocol described in the instruction manual. 1 ml of Tryzol was added for cell lysis, allowing it to rest for 5 minutes at 4°C. Subsequently, 200 µl of chloroform were added and centrifugation was carried out for 15 minutes at 12,000 rpm and 4°C. The supernatant was selected and diluted in 500 µl of isopropanol, homogenising the preparation and centrifuging at 12,000 rpm for 15 min and 4°C. Finally, 1 ml of cold 70% ethanol was added and the samples were centrifuged at 12,000 rpm for 5 minutes and 4°C. RNA concentration was quantified using spectrophotometric techniques (Nanodrop ND-1000). Complementary DNA (cDNA) synthesis was then carried out from 1 mg of RNA using Superscript^{®} VILO^{™} cDNA Synthesis kit (Invitrogen). For gene expression analysis, real-time quantitative PCR (RT-qPCR) was carried out. Each reaction used 5 µL of the cDNA dilution; 5 µL of mixture of primers in a proportion of 1:1 (10 µM sense primer, 10 µM antisense primer); 10 µL of SYBR Green (PowerUPTM SYBRTM Green Master Mix, Thermo Fisher) in a real-time thermocycler (LightCyclerâ480 System, Roche). The assays were performed in triplicate.

The *Ganoderma lucidum* carpophore extract at a concentration of 10 ng/ml increases IL-6 mRNA expression and reduces IL1-β and IL-8 expression with respect to untreated fibroblasts. The *Ganoderma lucidum* carpophore extract has a proinflammatory effect with respect to IL-6 (Table 1).

The *Hericium erinaceus* carpophore extract at a concentration of 10 ng/ml reduces IL1-β and IL-8 expression with respect to untreated fibroblasts. The *Hericium erinaceus* carpophore extract has a proinflammatory effect with respect to IL1-6 (Table 1).

**Table 1. IL1β, IL-6, and IL-8 mRNA expression in untreated fibroblasts (UT), fibroblasts treated with Ganoderma lucidum carpophore extract at a concentration of 10 ng/ml (F2), fibroblasts treated with Hericium erinaceus extract at a concentration of 10 ng/ml (F3), fibroblasts treated with a mixture of Ganoderma lucidum carpophore extract and Hericium erinaceus carpophore extract (F5) at a concentration of 10 ng/ml.**

| mRNA expression in fibroblasts | | | |
|---|---|---|---|
| Treatment | IL-1β | IL-6 | IL-8 |
| UT | 1.000 | 1.000 | 1.000 |
| F2 | 0.6689 | 1.4925 | 0.9909 |
| F3 | 0.7697 | 1.0465 | 0.8700 |
| F5 | 0.6820 | 0.9455 | 0.7076 |

Surprisingly, the combination of the *Ganoderma lucidum* carpophore extract with the *Hericium erinaceus* carpophore extract at a concentration of 10 ng/ml reduces the expression of different inflammatory cytokines in comparison to the extracts individually. Specifically, it has been observed that for IL-6 and IL-8, the combination of the *Ganoderma lucidum* carpophore extract and the *Hericium erinaceus* carpophore extract reduces the expression of these cytokines by 25% and 23.65%, respectively, in comparison to the use of the extracts individually. In fact, the combination of the *Ganoderma lucidum* carpophore extract and the *Hericium erinaceus* carpophore extract protects against the inflammatory effect induced by the use of the extracts individually in the case of IL-6.

The administration of the *Ganoderma lucidum* carpophore extract combined with the *Hericium erinaceus* carpophore extract significantly reduced the mRNA expression of the cytokines IL-1 β, IL-6, IL-8, particularly IL-8, in comparison to the administration of the fungal extracts individually. This combination is not expected to increase the anti-inflammatory effect of the extracts.

Cytokines are important signalling molecules that mediate communicative interactions both locally between different cell types within dermal tissues and at a distance between organs. Scientific studies indicate that the main cytokines in skin cell response to ionising radiation include IL (interleukin)-1β, IL-6 and the chemokines IL-8.

Various studies correlate cytokine gene expression in skin tissues with acute and chronic skin toxicity induced by radiation and chemotherapy processes (Müller and Meineke. Exp Hematol. 2007; 35(4 Suppl 1):96-104). In fact, the mechanism through which both treatments act is based on the induction of a generalised senescence in tumour cells, which contributes to persistent systemic and local tissue inflammation. Senescent cells secrete a series of inflammatory factors into the extracellular environment known as senescence-associated secretory phenotype (SASP). The secretion of SASP is beneficial in the short term as it allows the activation of the immune system, and thus favours the elimination of senescent cells from the tissue. However, in the long term, SASP generates an environment of prolonged inflammation that is sustained over time and complicates the elimination of tumour cells, even favouring their proliferative capacity. Therefore, inflammation is an inherent component of the chronic effects induced by radiation and chemotherapy, which suggests that the inhibition of inflammation, including the suppression of the acute effects of inflammatory cytokine gene expression, would reduce skin toxicity; therefore, a composition or combination comprising *Ganoderma lucidum* and *Hericium erinaceus* extracts can be used in the prevention and/or treatment of the side effects associated with radiotherapy and/or chemotherapy (Wyld et al. Cancers. 2020. 12(8): 2134; Krtolica et al. Biological Science. 2001. 98 (21) 12072-12077).

## Claims

1. A topical composition comprising an extract obtained from *Ganoderma lucidum* and an extract obtained from *Hericium erinaceus.*

2. The composition according to claim 1, wherein the extract obtained from *Ganoderma lucidum* is an extract obtained from the carpophore of *Ganoderma lucidum*; and wherein the *Hericium erinaceus* extract is an extract obtained from the carpophore of *Hericium erinaceus.*

3. The composition according to any one of claims 1 or 2, wherein the *G. lucidum* extract comprises β-glucans, ergosterol, and ganoderic acids; and wherein the *H. erinaceus* extract comprises β-glucans, γ-aminobutyric acid, ergosterol, and hericenone c.

4. The composition according to any one of claims 1 to 3, wherein the *G. lucidum* extract comprises:
- between 524 mg/g and 641 mg/g of β-glucans;
- between 376 µg/g and 460 µg/g of ergosterol; and
- between 4.30 µg/g and 5.30 µg/g of ganoderic acid.

5. The composition according to any one of claims 1 to 4, wherein the *H. erinaceus* extract comprises:
- between 220 mg/g and 268 mg/g of β-glucans;
- between 46 µg/g and 56.32 µg/g of γ-aminobutyric acid;
- between 313.39 µg/g and 382.80 µg/g of ergosterol; and
- between 0.18 µg/g and 0.22 µg/g of hericenone c.

6. The composition according to any one of claims 1 to 5, **characterised in that**:
- the *G. lucidum* extract is in an amount of between 0.005% and 25%, and
- the *H. erinaceus* extract is in an amount of between 0.005% and 25%,
wherein the % refers to the % m/m of each of the extracts with respect to the total composition.

7. The composition according to any one of claims 1 to 6, wherein said composition is in liquid or semisolid form.

8. The composition according to any one of claims 1 to 7, wherein the composition is in the form of a gel, cream, or ointment.

9. The composition according to any one of claims 1 to 8, wherein said composition is a pharmaceutical composition or a cosmetic composition.

10. The composition according to claim 9, wherein said composition further comprises a pharmaceutically or cosmetically acceptable vehicle and/or excipient.

11. A combination comprising an extract obtained from *Ganoderma lucidum* and an extract obtained from *Hericium erinaceus* for use as a medicament, wherein said medicinal product is administered topically.

12. A combination comprising an extract obtained from *Ganoderma lucidum* and an extract obtained from *Hericium erinaceus* for use in the treatment and/or prevention of the side effects of radiotherapy and/or chemotherapy on the skin of a subject, wherein said combination is administered topically.

13. The combination for use according to any one of claims 11 or 12, wherein the extract obtained from *Ganoderma lucidum* is an extract obtained from the carpophore of *Ganoderma lucidum*; and wherein the *Hericium erinaceus* extract is an extract obtained from the carpophore of *Hericium erinaceus.*

14. The combination for use according to any one of claims 12 or 13, wherein the side effect of radiotherapy and/or chemotherapy is selected from the list consisting of: fibrosis, inflammation, dermatitis, skin fragility, erosions in skin folds, loss of nails, and/or dry skin.

15. The combination for use according to any one of claims 11 to 14, wherein the *Ganoderma lucidum* extract and the *Hericium erinaceus* extract are administered simultaneously, separately, or sequentially.

16. The combination for use according to any one of claims 11 to 15, wherein the *Ganoderma lucidum* and *Hericium erinaceus* extracts are comprised in one and the same composition or in different compositions, preferably wherein the *Ganoderma lucidum* extract is in an amount of between 0.005% m/m and 25% m/m, and wherein the *Hericium erinaceus* extract is in an amount of between 0.005% m/m and 25%m/m, wherein the % refers to the % m/m of each of the extracts with respect to the total composition in which they are found.

17. The combination for use according to claim 16, wherein the composition or compositions are pharmaceutical composition or compositions.

18. The combination for use according to claim 17, wherein said composition or compositions further comprise a pharmaceutically acceptable vehicle and/or excipient.

19. The combination for use according to any one of claims 16 to 18, wherein said composition or compositions are in liquid or semisolid form.

20. The combination for use according to any one of claims 16 to 19, wherein the composition or compositions are formulated in the form of a gel, cream, or ointment.

21. The combination for use according to any one of claims 16 to 20, wherein the composition comprising the extract obtained from *Ganoderma lucidum* and the extract obtained from *Hericium erinaceus* is the composition according to any one of claims 1 to 10.

22. The combination for use according to any one of claims 12 to 21, wherein the subject has cancer, preferably breast cancer.

23. The combination for use according to any one of claims 12 to 22, wherein the subject is a human or a non-human mammal.

24. Non-therapeutic cosmetic use of a combination comprising an extract obtained from *Ganoderma lucidum* and an extract obtained from *Hericium erinaceus,* wherein said combination is administered topically to a subject.

25. Non-therapeutic cosmetic use according to claim 24, wherein the extract obtained from *Ganoderma lucidum* is an extract obtained from the carpophore of *Ganoderma lucidum*; and wherein the *Hericium erinaceus* extract is an extract obtained from the carpophore of *Hericium erinaceus.*

26. Non-therapeutic cosmetic use according to any one of claims 24 or 25, wherein the *Ganoderma lucidum* extract and the *Hericium erinaceus* extract are administered simultaneously, separately, or sequentially.

27. Non-therapeutic cosmetic use according to any one of claims 24 to 26, wherein the *Ganoderma lucidum* and *Hericium erinaceus* extracts are comprised in one and the same composition or in different compositions, preferably wherein the *Ganoderma lucidum* extract is in an amount of between 0.005% m/m and 25% m/m, and wherein the *Hericium erinaceus* extract is in an amount of between 0.005% m/m and 25%m/m, wherein the % refers to the % m/m of each of the extracts with respect to the total composition in which they are found.

28. Non-therapeutic cosmetic use according to claim 27, wherein the composition or compositions are cosmetic composition or compositions.

29. Non-therapeutic cosmetic use according to claim 28, wherein said composition or compositions further comprise a cosmetically acceptable vehicle and/or excipient.

30. Non-therapeutic cosmetic use according to any one of claims 27 to 29, wherein said composition or compositions are in liquid or semisolid form.

31. Non-therapeutic cosmetic use according to any one of claims 27 to 30, wherein the composition or compositions are formulated in the form of a gel, cream, or ointment.

32. Non-therapeutic cosmetic use according to any one of claims 27 to 31, wherein the composition comprising the extract obtained from *Ganoderma lucidum* and the extract obtained from *Hericium erinaceus* is the composition according to any one of claims 1 to 10.

33. Non-therapeutic cosmetic use according to any one of claims 24 to 32, wherein the subject is a human or a non-human mammal.
